# EUROPEAN PATENT APPLICATION

(11) **EP 3 828 284 A1**
(43) Date of publication of application: **02.06.2021**
(21) Application number: 20216686.4
(22) Date of filing: 14.03.2014
(51) Int. Cl.: C12Q 1/6806, C12N 15/10

(54) **ONE-STEP PROCEDURE FOR THE PURIFICATION OF NUCLEIC ACIDS**

(30) Priority: 15.03.2013 US 201361789622 P
(62) Divisional of application: 14765295.2
(71) Applicant: Abbott Molecular Inc., Des Plaines, IL 60018 (US)
(72) Inventor: GUNDLING, Gerard, J, Lake Forest, IL Illinois 60045 (US)
(74) Representative: Redhouse, Juliet Lauren

(57) **Abstract**

The present invention is a new and non-obvious method for the improved and simplified purification of nucleic acids.

## Description

### Background

Nucleic acid purification typically consists of a lysis step to release nucleic acids from
a sample, the binding of the nucleic acids to a solid matrix, the washing of the matrix to
remove contaminating substances and the elution of the nucleic acids from the matrix in a
buffer. The process is complex, particularly in the washing of the matrix in the removal of
contaminants. Solutions must be added to the matrix which will remove contaminants but
not the nucleic acids and then these solutions must be removed prior to the elution of the
nucleic acids from the matrix. If magnetic particles are used as the solid matrix, they
typically must be captured after the binding step, separated from the lysis-binding solution,
either by capture and removal of the particles from the solution or by immobilization of the
particles and removal of the lysis-binding solution, and then released into a wash solution.
Following the wash the particles must be captured again and separated from the wash
solution. See, e.g., Alderton, R. P., et al., Anal. Biochem. (1992) 201:166-169 and WO
91/00212. Some protocols require several wash steps using several different wash
solutions. After the particles have been washed, they must be re-suspended in an elution
buffer to release the nucleic acids from the particles. Further, these procedures are often
not selective for nucleic acids. Rather, a variety of solid and dissolved substances are
agglutinated as well.
Other protocols require the precipitation of the nucleic acid with, for example,
ethanol. The precipitated nucleic acid must then be washed and resolubilized.
Chromatographic procedures also exist for the isolation of nucleic acid (see, e.g., EP 0 658
164) but these procedures also require multiple steps and washes.
The prior art SCODA (Synchronous Coefficient of Drag Alteration) system by
Boreal Genomics, Inc. (Los Altos, CA) uses a pulsed electronic field to focus nucleic acids
into a point but this is typically pre-purified material that may be very dilute. The oil-gate
system developed by Kelso at Northwestern University (Sur, et al., Immiscible Phase
Nucleic Acid Purification Eliminates PCR Inhibitors with a Single Pass of Paramagnetic
Particle through a Hydrophobic Liquid, J. Mol. Diag. 2012 12(5):620-628) drags the particles
through oil to try to eliminate washing but the method resulted in substantial salt carry over
because the oil left a large droplet of lysis solution around the particles which resulted in a
great deal of salt carryover. Thus, additional processing was necessary when using this
system.

It can be seen that the methods for the isolation/purification of nucleic acids that are
considered state of the art have certain disadvantages. Such disadvantages relate to, e.g.,
purity, selectivity, recovery rate, laboratory safety and convenience, as well as to the speed
of the isolation/purification process. In other words, known prior art procedures require
multiple steps and often result in loss of target nucleic acid due to numerous steps and/or
alteration of target nucleic acid (for example, loss of modifying groups due to repeated harsh
treatment conditions).
Thus, the problem to be solved is to provide a simpler procedure for isolating nucleic
acids from a sample that will save time and reagents and help prevent the loss of target
and/or modification of the target during processing.

Summary of the Invention

The invention relates to the use of a gel to separate the lysis-binding solution from an
elution buffer. Particles are used to capture nucleic acids in the lysis-binding solution. The
particles are magnetic and are concentrated using a magnetic field. Specifically, the
particles are drawn by a magnetic field through a gel that removes contaminants, and the
concentrated particles are re-suspended in an elution buffer. The process is extremely
simple. The gel separates the lysis-binding solution from the elution buffer and limits or
eliminates mixing between the two. The movement of the particles through the gel removes
contaminants and salts. Contaminants are removed by the passage through the gel (i.e.,
they are "squeegeed" out). Salts are diluted out by passing through the gel. The particles
then pass directly into the elution buffer where the nucleic acids are released. The magnetic
particles can then be drawn back into the gel or separated from the elution buffer by
centrifugation or settling (gravity). This process has been shown to successfully purify HCV
viral RNA from a plasma sample using a real-time PCR assay to detect the hepatitis C virus
(HCV viral) RNA.
The present invention contemplates a method of removing lysis buffer components
from a nucleic acid sample, the method comprising: providing a specimen comprising or
suspected of comprising nucleic acids, contacting the specimen with one or more lysis
reagents, contacting the specimen with a solid substrate for a length of time and under
conditions suitable for nucleic acids in the specimen to bind to the solid substrate and
causing the solid substrate bound with nucleic acids, if nucleic acid is present in the
specimen, to pass through an aqueous-based separation gel. The present method is useful
when the lysis buffer contains salts in a concentration greater than about 1 molar. The solid

substrate may be a microparticle and the microparticle may be from about 0.1 nm to about 5
nm in diameter or from about 0.8 nm to about 5 nm in diameter. Further, the microparticle
may be magnetic.

The present invention further contemplates that the nucleic acids bound to the solid
substrate are contacted with an elution buffer after passing through the aqueous gel which
elutes nucleic acids from the solid substrate. Further still, the nucleic acids are not
precipitated in an organic solvent or the nucleic acids may be precipitated in an organic
solvent and the organic solvent may be ethanol or any other suitable organic solvent.

The present invention further contemplates that the nucleic acids bound to the solid
substrate are contacted with an enzyme after passing through the aqueous gel, either
before or after elution from the solid substrate. The enzyme may be a DNA polymerase or a
reverse transcriptase. Further still, the nucleic acid may be sequenced on the solid
substrate without dilution.

The present invention further contemplates that the nucleic acids bound to the solid
substrate may be contacted with bisulfite after passing through the aqueous gel such that
unmethylated cytosines are deaminated. The present invention further contemplates that at
least one nucleic base in the nucleic acid may have an epigenetic modification.

The present invention still further contemplates that the nucleic acid may be bound to
the solid substrate via an entity selected from the group consisting of nucleic acid
hybridization, an aptamer, an antibody, an antibody fragment, biotin, avidin, and
streptavidin.

The present invention contemplates a method for enriching nucleic acids from a
sample, the method comprising: providing; a sample suspected of comprising nucleic acids,
microparticles suitable for binding nucleic acids, and an aqueous-based separation gel,
contacting the sample with the microparticles for a length of time and under conditions
suitable for any nucleic acids in the sample to bind to the magnetic microparticles to create
loaded magnetic microparticles; drawing the loaded microparticles through the aqueous-
based separation gel with a magnetic field. The gel may be an agarose gel. The agarose
gel may be at a concentration of about 0.10 % to about 1.0 %. The separating gel may also
comprise one of more of ethanol and glycerol. The particles may also comprise a functional
group suitable for binding nucleic acid.

It is further contemplated that the microparticles may be magnetic and the magnetic
microparticles are, optionally at least partly coated with one or more of silica and glass. The
magnetic microparticles may be spheroid although any shape is contemplated as being
suitable in the present invention.

It is further contemplated that the sample may comprise lysis buffer.

Description of the Figures

Figure 1 (A – F) shows an exemplification of the method of the present invention as
described in the Examples.

Figure 2 shows (A) DNA amplification curves of target nucleotide sequences purified
by the methods of the present invention as detailed in Example 2 and (B) internal controls.

Figure 3 shows (A) DNA amplification curves of target nucleotide sequences purified
by the methods of the present invention as detailed in Example 2 and (B) internal controls.

Detailed Description of the Invention

The present methods are directed toward a one-step isolation and purification
method of nucleic acids. The method is simple and easily performed by one of ordinary skill
in the art. The method involves adding to a lysis solution (lysis solutions are known to those
of ordinary skill in the art) and a solid substrate that binds nucleic acid (for example, iron
oxide particles, magnetic particles,magnetic particles that are at least partly coated with
glass, silica or the like, etc.). Nucleic acids present will bind the particles. The particles are
then drawn through an aqueous gel (e.g., an agarose gel although any gel compatible with
nucleic acids, for example an SDS acrylamide gel, would be suitable) with, in the case of
magnetic particles, a magnetic source (i.e., a magnet) positioned outside of the assay
vessel. The action of drawing the particles through the gel removes (i.e., "squeegees") non-
soluble contaminants and dilutes soluble contaminants (e.g., salts). The particles enter an
elution buffer after leaving the gel. The nucleic acid is released in the elution buffer. The
magnetic particles can then be drawn back into the gel or allowed to settle out (or
centrifuged out after removal of the elution buffer from the gel surface). The method is
typically performed in a tube wherein the gel is layered over the lysis buffer and the elution
buffer is layered over the gel. Optionally, a non-eluting buffer can be layered over the gel
and the nucleic acids eluted from the particles at a later time, if desired. The elution buffer
may or may not comprise an organic solvent (such as ethanol).

Typical lysis buffers comprise salts. Salts typically found in lysis buffers include, but
are not limited to guanidinium thiocyanate (GITC) (typical range of 0.5 M to 4.0 M), NaCl
(typical range of up to 0.1 M and 10.5 M). Lysis buffers also typically contain a buffer (for
example, Tris-HCl; typical concentration of about 25 mM, about pH 7 to about pH 8, or other
suitable buffer known to one of ordinary skill in the art) and detergents (for example, NP-40,
Tween™, Triton™-X, polysorbates, deoxycholate, sodium deoxycholate and sodium dodecyl
sulfate (SDS); concentrations typically used range from about 0.1 % to about 2.0 %).
The present invention is not limited to any specific sample or sample type. For
example, sample materials may include bodily fluids including plasma, serum, blood, spinal
fluid, semen, vaginal fluids, sputum and saliva, cerebrospinal fluid, lymphatic fluid and
digestive fluids. Other sample materials may include isolated or enriched cell populations
and tissues. Samples may be fresh or fixed (preserved). Fixed samples may be embedded
(for example, paraffin embedded). Further, samples may be obtained from archeological
digs, i.e., prehistoric tissues such as bones may yield nucleic acids that can be enriched or
isolated by the methods of the present invention. Certain sample types may require
pretreatment to, for example, concentrate the sample (by, for example, centrifugation of
suspended cells) or break apart large sample sources (for example, grinding of bones or
digestive breakdown of tissues). Such pretreatments are primary to obtain starting materials
that are more easily workable by the methods of the present invention.
The gels used in the present invention may be at a concentration of about 0.1 % to
about 1.0 %, about 0.15 % to about 0.75 %, about 0.2 % to about 0.50 %.
In the present specification it is understood that the term "a nucleic acid" denotes at
least one nucleic acid. Furthermore, the term "a nucleic acid" also may indicate a mixture of
nucleic acids. The term "nucleic acid" encompasses RNA, DNA, or both. Further, as used
herein, "nucleic acid" or "NA" refers to both a deoxyribonucleic acid and a ribonucleic acid.
As used herein, "nucleic acid sequence" refers to the order or sequence of
deoxyribonucleotides or ribonucleotides along a strand. They may be natural or artificial
sequences and, in particular, genomic DNA (gDNA), complementary DNA (cDNA),
messenger RNA (mRNA), transfer RNA (tRNA), ribosomal RNA (rRNA), hybrid sequences
or synthetic or semisynthetic sequences or oligonucleotides which are modified or contain
modified bases. These nucleic acids may be of human, animal, plant, bacterial or viral
origin and the like. They may be obtained by any technique known to persons skilled in the
art, and in particular by cell lysis, the screening of libraries, by chemical synthesis or by
mixed methods including the chemical or enzymatic modification of sequences obtained by

the screening of libraries. They may be chemically modified, e.g. they may be pseudonucleic
acids (PNA), oligonucleotides modified by various chemical bonds (for example
phosphorothioate or methyl phosphonate), or alternatively oligonucleotides which are
functionalized, e.g., which are coupled with one or more molecules having distinct
characteristic properties.
The methods of the present invention may be altered to specifically isolate or enrich
either DNA or RNA. For example, the use non-coated iron oxide as a capture particle would
be selective for RNA. Silica particles or silica coated particles, for example, would be
specific for DNA. However, if you make the lysis buffer 33% ethanol, silica particles, for
example, will bind both DNA and RNA for total nucleic acids isolation or enrichment.
The term "substrate" denotes a substance which is substantially insoluble in an
aqueous solution and on which a nucleic acid in an aqueous solution (of high ionic strength)
can adsorb when the substance is added. The term "substrate" encompasses magnetically
attractable particles and magnetically attractable particles such as uncoated iron oxide or
coated with, for example, silica, glass, quartz or zeolites. However, the substrate need not
be magnetic. Further, the particles may comprise functional groups suitable for binding
nucleic acids. Examples of such functional groups include but are not limited to proteins (or
functional portions thereof), antibodies (or functional portions thereof), chemical linkers (e.g.,
avidin, streptavidin, via nucleic acid hybridization, an aptamer, biotin, etc.). Thus, the
nucleic acid need not be bound directly to the substrate but may be bound via one or more
entities, as described herein or as are known to one of ordinary skill in the art. Further, non-
magnetic particles may be caused to pass though the aqueous-based gel by, for example,
centrifugation (e.g., low speed centrifugation). In this embodiment, it is contemplated that
the elution buffer (or saline, etc.) would be at bottom of the tube with the gel layered over the
elution buffer and the lysis buffer on top of the gel. The force of the centrifugation would pull
the particles down though the gel into the elution buffer. In another embodiment, the tube is
set up as with using a magnetic force (lysis buffer on bottom, elution buffer on top) and the
tube is capped and inverted before centrifugation. Further, other means for moving the
microparticles though the gel are contemplated. For example, charged particles may be
drawn through the gel by electrophoretic-type methods. Further still, if the particles are
allowed to settle (or centrifuged to the level of the gel) and the lysis buffer is removed, the
microparticles can be pushed through the gel with a device similar to a French press.
The substrate can be of any shape including, but not limited to, spherical, elliptical,
oblong, rod shaped, spiral and amorphous. The substrate of the present invention is not

limited by size but is preferably about 0.2 mm or less, 0.02 mm or less, 2.0 μm or less, 200
nm or less, 20 nm or less and 2 nm or less in the largest diameter. The substrate of the
present invention may also be about 0.1 nm to about 5 nm and may be about 0.8 nm to
about 5 nm in diameter. Further, it is understood that a substrate, when dispersed in a liquid
phase such as an aqueous solution, may produce a suspension or may settle out of the
solution.
The terms "high ionic strength" and "high concentration" mean the ionic strength or
concentration in an aqueous solution that results from dissolved salts in concentrations
equal to or greater than about 1 M. Typically salts are present in the aqueous solution in
concentrations of 1 to 10 M. More typical are chaotropic salts in concentrations of 1 to 8 M.
In a preferred embodiment, the lysis buffer of the present invention has a salt concentration
of greater than about 1 M.
The terms "low ionic strength" and "low concentration" mean the ionic strength or
concentration in an aqueous solution that results from dissolved salts in concentrations less
than about 1 M.
The terms "magnetic" and "magnetic particle(s)" shall refer to objects made from a
material that is magnetized and/or creates its own persistent magnetic field. Although any
suitable material as is known to one of ordinary skill in the art is contemplated for use in this
invention, iron-based materials (e.g., iron oxide) or material in which comprise iron oxide are
most frequently contemplated. In the present invention the magnetic particles
(multifunctional magnetic particles; MMP) form a substrate and, while not limited by size, are
preferably about 0.2 mm or less, 0.02 mm or less, 2.0 μm or less, 200 nm or less, 20 nm or
less and 2 nm or less in the largest diameter. In a preferred embodiment of the present
invention, the magnetic particles of the present invention are coated at least partly with
glass, silica or one or more substances known to one of ordinary skill in the art to bind
nucleic acids. Micro- and nano-sized magnetic particles are commercially available to one
of ordinary skill in the art (e.g., Promega Corp., Madison, WI; Life Technologies, Grand Isle,
NY; Bangs Laboratories, Fishers, IN). Further, particles can be synthesized in the lab by
one of ordinary skill in the art, see, for example, Starmans LW, et al., Iron Oxide
Nanoparticle-Micelles (ION-Micelles) for Sensitive (Molecular) Magnetic Particle Imaging
and Magnetic Resonance Imaging, PLoS One, Epub 2013 Feb 20.;8(2)e57335; Heitsch,
Andrew T., et al., Multifunctional Particles: Magnetic Nanocrystals and Gold Nanorods
Coated with Fluorescent Dye-Doped Silica Shells, Solid State Chem. 2008 July; 181(7):
1590–1599.

Methods and reagents for lysing cells and tissues for the extraction of nucleic acids
are well known by those or ordinary skill in the art and any method that does not destroy the
nucleic acids of the sample are suitable for use by the present invention. Further, nucleic
acids suitable for isolation and purification by the present method may be obtained from
fixed samples (e.g., formalin or glutaraldehyde fixed samples), embedded samples (e.g.,
paraffin embedded samples) and from reaction vessels containing solutions or suspensions
of synthesized nucleic acids.
Nucleic acids enriched, isolated or purified by the methods of the present invention
may be used in any conventional method known to those of ordinary skill in the art because
the methods of the present invention do not alter the nucleic acids in any way that may be
detrimental to their subsequent use. For example, the nucleic acids may be sequenced,
amplified by PCR, used in expression vectors, etc. In this regard, the nucleic acids may be
contacted with enzymes such as, for example, a DNA polymerase or a reverse transcriptase
after passing through the aqueous gel. Further, this invention contemplates that the nucleic
acid is sequenced on the solid substrate without dilution. Further, this invention
contemplates that the nucleic acids bound to the solid substrate are contacted with bisulfite
after passing through the aqueous gel such that unmethylated cytosines are deaminated.
Further, this invention contemplates that at least one nucleic base in the nucleic acid has an
epigenetic modification.

Kits
The present invention also contemplates kits wherein the kits contain gel solutions
and/or gel ingredients (as exemplified below or equivalents as could be determined by one
of ordinary skill in the art without undue experimentation), magnetic particles suitable for
binding nucleic acids and instructions. Optionally, vials, reaction solutions and other items
could be provided in a kit of the present invention for specific uses (e.g., the isolation of
RNAs) and/or to aid the user with regard to performing the procedure.
All patents, patent application publications, journal articles, textbooks, and other
publications mentioned in the specification are indicative of the level of skill of those in the
art to which the disclosure pertains. All such publications are incorporated herein by
reference to the same extent as if each individual publication were specifically and
individually indicated to be incorporated by reference.
The invention illustratively described herein may be suitably practiced in the absence
of any element(s) or limitation(s), which is/are not specifically disclosed herein. Thus, for
example, each instance herein of any of the terms "comprising," "consisting essentially of,"

and "consisting of" may be replaced with either of the other two terms. Likewise, the
singular forms "a," "an," and "the" include plural references unless the context clearly
dictates otherwise. Thus, for example, references to "the method" includes one or more
methods and/or steps of the type, which are described herein and/or which will become
apparent to those ordinarily skilled in the art upon reading the disclosure.
The terms and expressions, which have been employed, are used as terms of
description and not of limitation. In this regard, where certain terms are defined under
herein and are otherwise defined, described, or discussed elsewhere in the "Detailed
Description" or elsewhere in this specification, all such definitions, descriptions and
discussions are intended to be attributed to such terms. There also is no intention in the use
of such terms and expressions of excluding any equivalents of the features shown and
described or portions thereof.
It is recognized that various modifications are possible within the scope of the
claimed invention. Thus, it should be understood that, although the present invention has
been specifically disclosed in the context of preferred embodiments and optional features,
those skilled in the art may resort to modifications and variations of the concepts disclosed
herein. Such modifications and variations are considered to be within the scope of the
invention as defined by the appended claims.

Exemplification

Example 1
The concept of the present invention is that nucleic acids can be easily purified from,
for example, cell lysates or other cellular sample, by binding the nucleic acid to magnetic
particles and then drawing the particles though a viscous, aqueous gel or gel solution. The
first test was to see if the gel can reduce the salt level in a nucleic acid-free sample.
A 1 % gel was made as follows. 50 ml of water (DNase and RNase free water) was
mixed with 0.5 g Agarose-LE (Ambion #9040, although any commercially available agarose
should be workable). The agarose was melted in the water in a microwave oven (although
any suitable heat source will work) to make a 1 % agarose solution and placed on a temp
block (i.e., a heat block) set at 55 °C to keep the gel melted.
The lysis-ethanol solution was made as using 70 ml of a guanidine thiocyanate
(GITC) containing lysis buffer (the lysis buffer having greater than 3M GITC, greater than 1

% non-ionic detergent and with a pH above 7.0; or any lysis buffer suitable for the tissue or
sample being processed that preserves nucleic acids) and 35 ml of 95% ethanol.
1 ml of the lysis buffer-ethanol solution was added to a 12 mm X 75 mm
polypropylene tube along with 0.5 ml of water to represent a sample addition. 50 microliters
of silica coated magnetic microparticles (Abbott laboratories, Abbott Park, Il,
mMicroparticlesDNA) were also added to the lysis mixture.
2 ml of the melted agarose was gently floated on top of the lysis buffer and let cool to
set. After hardening, the surface of the gel was washed to remove any residual guanidine
that may have gotten on top of the gel by adding 500 microliters of water (above) to the
surface of the gel and then removing the wash. 200 microliters of water (above) was then
added to the top of the gel to represent elution buffer.
The magnetic particles (MMP) were captured with a magnet by placing a magnet on
the side of the tube in the region of the lysis buffer. An alternative approach is to place a
greater magnetic source directly above the tube. The magnet was then slowly drawn up the
side of the tube with the magnetic field pulling the magnetic particles along. The magnetic
particles smeared out somewhat while passing through the gel. The collected particles were
on the side of the tube and as the bolus of particles moved up the tube the gel was
displaced by the particles. A channel was formed where the particles moved through the
gel. It may be possible to position a magnet such that the particles would be drawn up
directly through the center of the gel but the position of the magnet does not seem to matter
based on the results obtained.
The magnetic particles were moved through the gel layer to the water layer and
mixed back and forth by moving the magnet from one side to the other. This was done
several times to mix the particles in the water. The particles were then captured by placing
the magnet on the side of the tube and drawn back into the gel such that they were removed
from the top water layer. See, Figure 1 A – F. The water layer was removed and placed
into a microfuge tube. Absorption readings were taken at A 230, 260, 280 and 400 on a
Beckman DU-640 spectrophotometer although any commercial spectrophotometer will work.
The instrument was blanked with water and the samples were read.
Readings taken at A 230. Undiluted sample gave a reading of 2.8194 which is too
high for an accurate reading for this particular spectrophotometer. The sample was diluted
1/40 with water and reread. The reading was 1.7268 with the 40 X dilution. A factor of 0.6 X
was used to convert A230 to mM GITC. 1.7268 X 40 X 0.6 = 41.4 mM GITC. This
concentration of GITC in the sample would be acceptable. Several assays have been

tested and show a tolerance to GITC up to 50 mM in the reaction. The assays of interest
use and 1:1 sample volume:assay mix ratio and the 41.4 mM GITC in the sample would
equal about 20 mM GITC in an assay.
The test was repeated and this time the MMP were drawn up through the gel while
rotating the gel increasing the distance that the particles traveled. Spectrophotometer
measurement were made using a 1/50 dilution of the sample in water. The absorbance at
A230 was 0.8792. 0.8792 X 50 X 0.6 = 26.4 mM GITC in the sample which is an acceptable
range. This method was next tested with samples containing nucleic acid.

Example 2
In this experiment a known quantity of nucleic acid was used in the sample. The
nucleic acids tested were the Calibrator B from the Abbott RealTime™ HCV assay and the
HCV Internal Control used in the assay extractions. The Calibrator B is an RNA sample
containing HCV sequences at a concentration of approximately 10,000,000 Iu/ml
(international units per milliliter) in human plasma. The internal control is also an RNA
molecule used to test the extraction performance of the sample preparation systems used to
extract HCV for testing. The presence of glycerol and ethanol in the gel was also tested to
determine if they might have any effect on the extraction. The presence of ethanol may help
retain the nucleic acids on the particles and the glycerol may change the density of the gel.
The gels were made up as follows using Ambion Agarose-LE #9040 lot 064P80B.
Three reagent bottles with 25 ml of water in each were used to make the different gels. The
water was DNase and RNase free water. Agarose (Ambion Agarose-LE #9040 lot 064P80B)
was added to each bottle as follows.

0.05 gm agarose to bottle 1 to make a 0.2 % gel.
0.10 gm agarose to bottle 2 to make a 0.4 % gel.
0.15 gm agarose to bottle 3 to make a 0.6 % gel.

The agarose was melted in the microwave and then the bottles were placed on top of
the temp block heated to 55 °C to keep melted.
The Hepatitis C viral Calibrator B (Abbott Laboratories, Abbott Park, IL; this is a
known quantity of HCV nucleic acid in a negative human serum) and Internal Control
(Abbott Laboratories, Abbott Park, IL) was first extracted with the lysis-buffer ethanol mix

and captured on magnetic particles as follows. The lysis ethanol mix discussed in Example
1 was used as the lysis buffer.

The lysis mix contained
10 ml of the lysis ethanol mix
5 ml of HCV Calibrator B
350 µl of HCV Internal Control
500 µl of MMP (multifunctional magnetic particles)

The mix was put on a rocker plate at room temperature for 12 minutes.

During the lysate incubation, nine 15 ml tubes were set up as below.
Tube | Agarose | water | ethanol | glycerol | Final Agarose concentration
1 | 4 ml of 0.2% | 1 ml | 0 | 0 | 0.16%
2 | 4 ml of 0.4% | 1 ml | 0 | 0 | 0.32%
3 | 4 ml of 0.6% | 1 ml | 0 | 0 | 0.48%
4 | 4 ml of 0.2% | 0 | 1 ml | 0 | 0.16%
5 | 4 ml of 0.4% | 0 | 1 ml | 0 | 0.32%
6 | 4 ml of 0.6% | 0 | 1 ml | 0 | 0.48%
7 | 9 ml of 0.2% | 0 | 0 | 1 ml | 0.18%
8 | 9 ml of 0.4% | 0 | 0 | 1 ml | 0.36%
9 | 9 ml of 0.6% | 0 | 0 | 1 ml | 0.54%

The glycerol content was set at 10%. At 20% glycerol, the agarose was denser than the
lysis buffer and sank to the bottom of the tube.

After the lysis incubation was complete, the samples were extracted using the gel
technique as follows. 1.5 ml of the lysate was put into each of 9 tubes (12 mm X 75 mm
polypropylene). 2 ml of each agarose mix was gently put on top and let harden (about 4 °C
for ~10 minutes). 1 ml water was out on top of all tubes to remove any trace of lysis. The
condition with the lowest amount of agarose and ethanol started to float above the water
wash and the sample (#2) was not further tested. The water was removed from the top of
the gels and 200 µl of water was added to the top of all gels for elution. The magnetic
particles were captured on the side of the tube with a magnet and drawn up through the gel

to elution buffer as described in example 1. The magnetic particles were then moved the
particles back and forth in the elution buffer about 20 times using the magnet to draw them
from one side of the tube to the other. The particles were drawn back into the gel as
described above. The elution buffer was then removed and put into a microfuge tube.

From each condition a 10 µl sample was taken and dilute with 490 µl of water. The
absorption was read in a spectrophotometer (Beckman DU-640) at 230 nm to determine
GITC carryover as described in example 1.

The instrument was blanked with water and then the samples were read.

Sample | A230 | mM GITC | Dilution | Final GITC (mM)
1 | 0.3693 | 0.210919 | 50 | 10.54595
2 | not tested
3 | 0.4904 | 0.282627 | 50 | 14.13134
4 | 0.3316 | 0.188595 | 50 | 9.429773
5 | 0.4461 | 0.256395 | 50 | 12.81975
6 | 0.8110 | 0.472466 | 50 | 23.62328
7 | 0.5354 | 0.309273 | 50 | 15.46364
8 | 0.5223 | 0.301516 | 50 | 15.07579
9 | 0.5418 | 0.313063 | 50 | 15.65313

All the samples have GITC concentrations below 25 mM.
The samples were then tested for the presence of the HCV and Internal Control
sequences using the Abbott RealTime™ HCV assay. A mastermix was made using the
three components of the Abbott RealTime™ HCV assay code 4J86-90. The 320 microliters
of Activator and 948 microliters of Oligo mix were added to the preloaded enzyme bottle and
gently mixed by pipetting. 25 microliters of the mastermix was then mixed with 25
microliters of each eluated sample in a well of a PCR plate. 25 microliters of this mixture
was then transferred to a Cepheid Tube and cycled using a Cepheid (Sunnyvale, CA)
SmartCycler® with the following program, below.

Temp C | Time seconds

Step 1 for 1cycle
55 | 1200

Step 2 for 4 cycles
Temp | Time
95 | 20
46 | 30
68 | 20

Step 3 for 6 cycles
Temp | Time
92 | 20
60 | 30
68 | 20

Step 4 for 37 cycles
Temp | Time
90 | 20
56 | 40
68 | 20
35 | 40

Each sample was then read.

The amplification curves are below and show that both HCV and the internal control were
amplified from the eluates. Figure 2 shows graphs of the amplification curves (A) and
internal controls (B).

The second 25 microliters of each of the assay mixes were also amplified using a different
program, also in the Cepheid SmartCycler.

Temp C | Time seconds
Step 1 for 1 cycle
Temp | Time
55 | 1800

Step 2 for 4 cycles
Temp | Time
95 | 40
46 | 30
68 | 20

Step 3 for 6 cycles
Temp | Time
92 | 40

60 | 30
68 | 20

Step 4 for 37 cycles
Temp | Time
90 | 30
56 | 40
68 | 20
35 | 40

Each sample was read.

Figure 3 shows graphs of the amplification curves (A) and internal controls (B).

These experiments demonstrate that it is possible to capture HCV on magnetic
microparticles, draw the particles up through a gel, which removes high levels of GITC and
other contaminants, into an elution buffer, elute the sample and use the eluted sample in a
PCR assay without further purification of the nucleic acid. The extraction protocol did not
use any separate washing steps.

Preferred embodiments of the present invention are described below and are
referred to as embodiments E1 to E 24.

E1) A method of removing lysis buffer components from a nucleic acid sample, the method
comprising
a) providing a specimen comprising or suspected of comprising nucleic acids,
b) contacting the specimen with one or more lysis reagents,
c) contacting the specimen with a solid substrate for a length of time and under
conditions suitable for nucleic acids in the specimen to bind to the solid substrate
and
d) causing the solid substrate bound with nucleic acids, if nucleic acid is present in the
specimen, to pass through an aqueous-based separation gel.

E2) The method of E 1, wherein the lysis buffer contains salts in a concentration greater
than about 1 molar.

E3) The method of E 1, wherein the solid substrate is a microparticle.

E4) The method of E 3, wherein the solid substrate is a microparticle from about 0.1 nm
to about 5 nm in diameter.

E5) The method of E 4, wherein the solid substrate is a microparticle from about 0.8 nm
to about 5 nm in diameter.

E6) The method of E 3, wherein the microparticle is magnetic.

E 7) The method of E 1, wherein the nucleic acids bound to the solid substrate are
contacted with an elution buffer after passing through the aqueous gel which elutes
nucleic acids from the solid substrate.

E8) The method of E 7, wherein the method does not include precipitation of the nucleic
acids in an organic solvent.

E9) The method of E 7, wherein the method does include precipitation of the nucleic
acids in an organic solvent and the organic solvent is ethanol.

E10) The method of E 3, wherein the nucleic acids bound to the solid substrate are
contacted with an enzyme after passing through the aqueous gel.

E11) The method of E 10, wherein the enzyme is a DNA polymerase or a reverse
transcriptase.

E12) The method of E 10, wherein the nucleic acid is sequenced on the solid substrate
without dilution.

E 13) The method of E 3, wherein the nucleic acids bound to the solid substrate are
contacted with bisulfite after passing through the aqueous gel such that unmethylated
cytosines are deaminated.

E14) The method of E 13, further comprising determining whether at least one nucleic
base in the nucleic acid has an epigenetic modification.

E 15) The method of E 1, wherein the nucleic acid is bound to the solid substrate via an
entity selected from the group consisting of nucleic acid hybridization, an aptamer, an
antibody, an antibody fragment, biotin, avidin, and streptavidin.

E16) A method for enriching nucleic acids from a sample, the method comprising:
a) providing;
i) a sample suspected of comprising nucleic acids,
ii) microparticles suitable for binding nucleic acids, and
iii) an aqueous-based separation gel,
b) contacting the sample with the microparticles for a length of time and under
conditions suitable for any nucleic acids in the sample to bind to the magnetic
microparticles to create loaded magnetic microparticles;
c) drawing the loaded microparticles through the aqueous-based separation gel with a
magnetic field.

E17) The method of E 16, wherein said separating gel comprises agarose.

E18) The method of E 17, wherein said agarose is at a concentration of about 0.10 % to
about 1.0 %.

E19) The method of E 17, wherein said separating gel also comprises one of more of
ethanol and glycerol.

E20) The method of E 16, wherein said particles comprise a functional group suitable for
binding nucleic acid.

E21) The method of E 16, wherein said microparticles are magnetic.

E22) The method of E 21, wherein said magnetic microparticles are at least partly coated
with one or more of silica and glass.

E 23) The method of E 21, wherein said magnetic microparticles are spheroid.

E 24) The method of E 16, wherein said sample comprises lysis buffer.

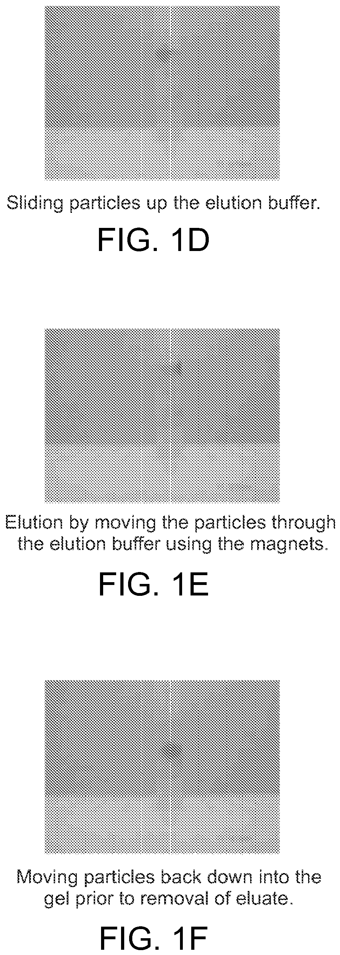

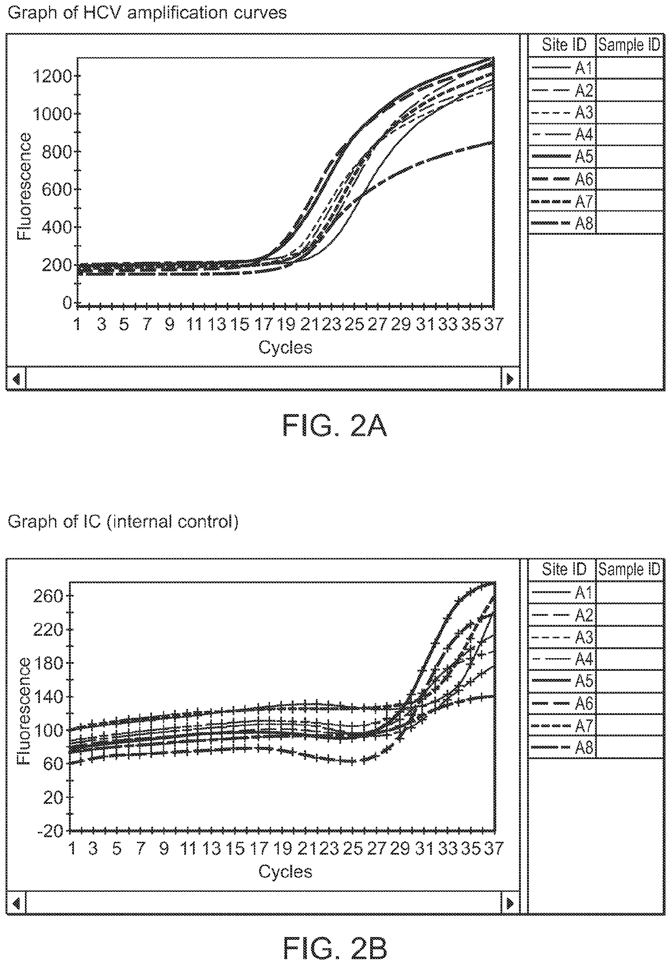

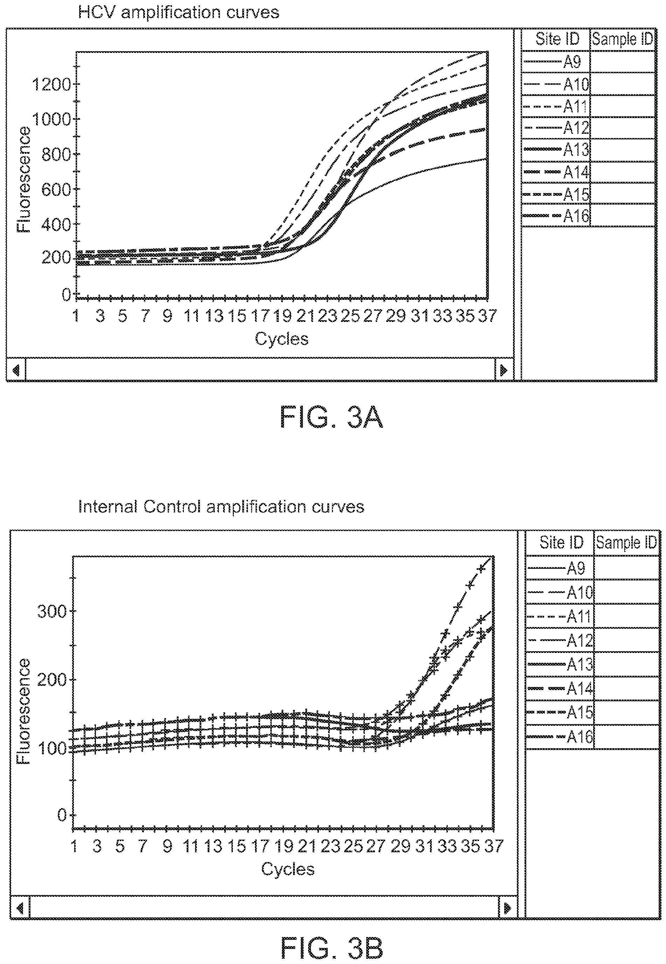

## Claims

1. A method for enriching nucleic acids from a sample, the method comprising:
a) providing
i) a sample suspected of comprising nucleic acids,
ii) magnetic microparticles suitable for binding nucleic acids, and
iii) an aqueous-based separation gel;
b) contacting the sample with the magnetic microparticles for a length of time and under conditions suitable for any nucleic acids in the sample to bind to the magnetic microparticles to create loaded magnetic microparticles; and
c) drawing the loaded microparticles through the aqueous-based separation gel with a magnetic field.

2. The method of Claim 1, wherein the separation gel comprises agarose, optionally wherein the agarose is at a concentration of about 0.10 % to about 1.0 %.

3. The method of Claim 1 or 2, wherein the separation gel further comprises one of more of ethanol and glycerol.

4. The method of any one of the preceding claims, wherein the particles comprise a functional group suitable for binding nucleic acid, optionally wherein the functional group is selected from the group consisting of a protein, a protein fragment, an antibody, an antibody fragment, or a chemical linker, e.g. avidin, streptavidin, nucleic acid hybridization, an aptamer or biotin.

5. The method of any one of the preceding claims, wherein the magnetic microparticles are at least partly coated with one or more of silica and glass.

6. The method of any one of the preceding claims, wherein the magnetic microparticles are spheroid.

7. The method of any one of the preceding claims, wherein the sample comprises lysis buffer, optionally wherein the lysis buffer contains salts in a concentration greater than about 1 molar.

8. The method of any one of the preceding claims, wherein (c) comprises drawing the loaded microparticles through the aqueous-based separation gel with a magnetic field into a buffer.

9. The method of Claim 8, wherein the buffer is an elution buffer or a non-elution buffer.

10. The method of Claim 9, wherein the separation gel is layered over the lysis buffer and the elution buffer or the non-elution buffer is layered over the aqueous-based separation gel.

11. The method of Claim 9 or 10, wherein:
(a) the elution buffer comprises an organic solvent, optionally wherein the organic solvent is ethanol; or
(b) the elution buffer does not comprise an organic solvent.

12. The method of any one of the preceding claims, wherein the magnetic microparticles are about 0.2 mm or less, 0.02 mm or less, 2.0 µm or less, 200 nm or less, 20 nm or less or 2 nm or less in the largest diameter.

13. The method of any one of the preceding claims, wherein the nucleic acids bound to the magnetic microparticles are contacted with an enzyme after passing through the aqueous gel, optionally wherein enzyme is a DNA polymerase or a reverse transcriptase.

14. The method of any one of the preceding claims, wherein the nucleic acid is sequenced on the magnetic microparticles without dilution.

15. The method of any one of the preceding claims, wherein the nucleic acids bound to the magnetic microparticles are contacted with bisulfite after passing through the aqueous gel such that unmethylated cytosines are deaminated, optionally further comprising determining whether at least one nucleic base in the nucleic acid has an epigenetic modification.
